(19) Europäisches Patentamt
European Patent Office
Office européen des brevets

(11) Publication number: **0 061 206**
**B1**

(12) **EUROPEAN PATENT SPECIFICATION**

(45) Date of publication of patent specification: **19.09.84**

(51) Int. Cl.[3]: **C 07 D 499/42,**
C 07 D 499/12, C 07 D 499/32
// C07D317/40

(21) Application number: **82103950.0**

(22) Date of filing: **29.04.81**

(60) Publication number of the earlier application in accordance with Art. 76 EPC: **0 039 086**

(54) **6-Aminopenicillanic acid esters and their use for producing new ampicillin esters.**

(30) Priority: **30.04.80 JP 58510/80**
**22.05.80 JP 68444/80**

(43) Date of publication of application:
**29.09.82 Bulletin 82/39**

(45) Publication of the grant of the patent:
**19.09.84 Bulletin 84/38**

(84) Designated Contracting States:
**AT BE CH DE FR GB IT LI LU NL SE**

(56) References cited:
**GB-A-1 377 661**
**GB-A-1 390 754**

(73) Proprietor: **KANEBO LTD.**
**17-4 Sumida 5-chome Sumida-ku**
**Tokyo 131 (JP)**

(72) Inventor: **Sakamoto, Fumio**
**12-32, Shiginonishi 5-chome Jyoto-ku**
**Osaka-shi Osaka-fu (JP)**
Inventor: **Ikeda, Shoji**
**Venus Heights No.605 2-1, Mizuo**
**Ibaraki-shi Osaka-fu (JP)**
Inventor: **Tsukamoto, Goro**
**2-8, Toneyama 2-chome**
**Toyonaka-shi Osaka-fu (JP)**
Inventor: **Utsumi, Isamu**
**47, Okazakitenno-cho Sakyo-ku**
**Kyoto-shi Kyoto-fu (JP)**

(74) Representative: **Kraus, Walter, Dr.**
**Patentanwälte Kraus & Weisert Irmgardstrasse 15**
**D-8000 München 71 (DE)**

**EP 0 061 206 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This application is a divisional application of 81 103 231.7. The present invention relates to novel 6-aminopenicillanic acid esters and to their use for producing new Ampicillin esters.

Ampicillin (aminobenzylpenicillin) obtained by acylating the amino group of 6-aminopenicillanic acid (6—APA) with $\alpha$-aminophenylacetic acid is a synthetic penicillin which is effective in oral administration. Absorption of Ampicillin from the digestive tract, however, is not sufficient, and this necessarily leads to administration of large dosages for obtaining the required concentration in blood, which in turn causes increased side-effects.

To remove such a defect of Ampicillin, an attempt was made to convert Ampicillin to an ester-type derivative thereby improving its absorption from the intestinal tract. For example, Ampicillin pivaloyloxymethyl ester (Pivampicillin; see GB—A—1,215,812), and Ampicillin phthalidyl ester (Talampicillin; see GB—A—1,364,672) gives comparable blood Ampicillin concentrations in oral administration to those obtained by intramuscular administration.

It is the object of this invention to provide a novel precursor for production of novel Ampicillin esters which are more stable in gastric and intestinal juices, have better absorption from the intestinal tract, maintain a high concentration in blood over longer periods of time than known Ampicillin esters such as Talampicillin and have low toxicity.

This object and advantage is achieved by 6-aminopenicillanic acid esters of the general formula I

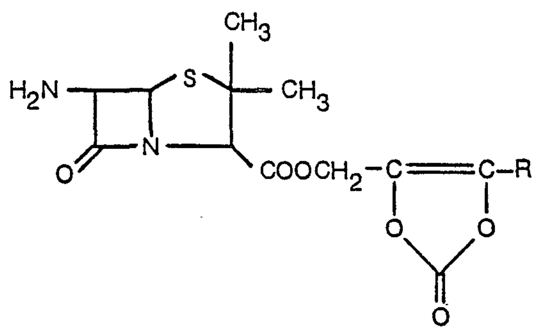

(I)

wherein R represents a methyl group or a phenyl group,
or its acid addition salt.

The inventive 6-aminopenicillanic acids of general formula I include
(5-methyl-2-oxo-1,3-dioxolen-4-yl)methyl 6-aminopenicillanate,
(2-oxo-5-phenyl-1,3-dioxolen-4-yl)methyl 6-aminopenicillanate,
and
acid addition salts of these esters.

The inventive compounds of general formula I can be produced by reacting 6-aminopenicillanic acid or its salt at the carboxyl group with a compound of the general formula III

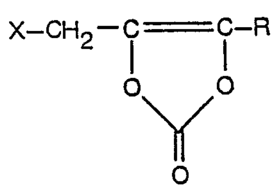

(III)

wherein R is as defined above, and X represents a halogen atom;
or by reacting 6-protected aminopenicillanic acid or its salt at the carboxyl group with the compound of general formula III and then converting the protected amino group of the reaction product to an amino group.

The former can be performed preferably by reacting 6-aminopenicillanic acid or its salt at the carboxyl group with an equimolar amount, or a molar excess, of the compound of general formula III in an inert organic solvent such as dimethylformamide, dimethyl sulfoxide, tetrahydrofuran, dioxane or acetone in the optional presence of a base (when 6-aminopenicillanic acid is used, the presence of a base is preferred) at a temperature of from 0°C to room temperature.

The latter can be performed preferably by reacting 6-protected aminopenicillanic acid such as 6-aminopenicillanic acid having the amino group at the 6-position protected with an acyl group or trityl group, or 6-aminopenicillanic acid having the amino group at the 6-position protected as a Schiff base, or its salt at the carboxyl group, for example 6-phenylacetylaminopenicillanic acid (benzylpenicillin), with the compound of general formula III under the same conditions as in the first-mentioned process, thereafter reacting the resulting 6-protected aminopenicillanic acid ester with phosphorus pentachloride and a lower alcohol such as methanol, ethanol or propanol at the temperature of dry ice-acetone in the presence of a basic compound such as N-methylmorpholine, quinoline and triethylamine, and thereafter causing water to act on the resulting imino ether to hydrolyze it.

According to a further aspect, this invention is directed to the use of the 6-aminopenicillanic acid esters as defined above for the production of Ampicillin esters of the general formula II

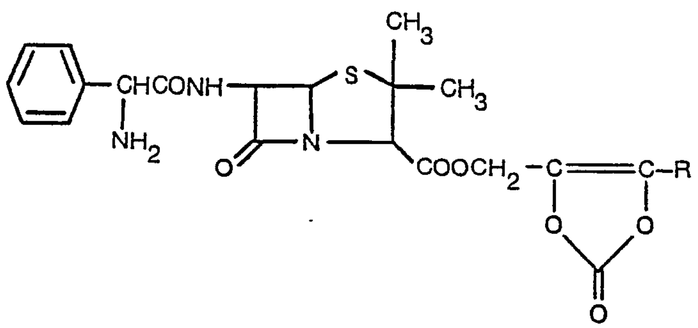

(II)

wherein R represents a methyl group or a phenyl group, or its acid addition salt.

Using the inventive 6-aminopenicillanic acid esters I, the Ampicillin esters of general formula II are produced by reacting an inventive 6-aminopenicillanic acid ester with a carboxylic acid of the general formula IV

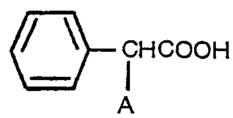

(IV)

wherein A represents a protected amino group, or a group convertible to an amino group, or its reactive derivative at the carboxyl group; thereafter, if required, when the resulting compound has the protected amino group or the group convertible to an amino group, eliminating the protective group from the protected amino group, or converting said convertible group to an amino group, and if further required, converting the product to an acid addition salt thereof.

In this process, the compound of general formula I or its acid addition salt may be a mineral acid salt or an organic acid salt, for example such a mineral acid salt as a hydrochloride or hydrobromide, or such an organic salt as a p-toluenesulfonate.

Acid halides, acid anhydrides and mixed acid anhydrides are preferably used as the reactive derivative of the carboxylic acid of general formula IV.

The reaction of the compound of general formula I or its acid addition salt with the carboxylic acid of general formula IV is carried out in the presence of a dehydrocondensing agent such as dicyclohexylcarbodiimide (DCC) or a mixture of DCC and 1-hydroxybenzotriazole, preferably in a solvent consisting substantially of an aprotic inert organic solvent such as dimethylformamide, dimethyl sulfoxide, methylene chloride, dioxane and tetrahydrofuran at a temperature of not more than 50°C.

The reaction of the compound of general formula I or its acid addition salt with the reactive derivative of the carboxylic acid of general formula IV is carried out preferably in a solvent consisting substantially of an aprotic inert organic solvent such as dimethylformamide, dimethyl sulfoxide, methylene chloride, dioxane, tetrahydrofuran and acetone at a temperature of not more than 50°C. When an acid addition salt of the compound expressed by general formula I is used, the reaction is preferably carried out in the presence of a base such as triethylamine.

The reactive derivative of the carboxylic acid of general formula IV used in the above reaction is preferably an acid halide such as an acid chloride when the group A in general formula IV is a protected amino group in the form of a salt with a mineral acid. The acid halide of the compound of general formula IV having such a group A can be conveniently produced by treating the compound of general formula IV having such a group A with a halogenating agent such as thionyl chloride, phosgene or phosphorus pentachloride because such group A is stable to acids.

The reactive derivative of the compound of general formula IV in which the group A is a protected amino group in the form of a Schiff base or an enamine group is preferably an acid anhydride or mixed acid anhydride. This reactive derivative can be produced conveniently by treating a salt, such as a trialkylamine salt, of the carboxylic acid of general formula IV in which the group A is such a protected amino group, with, for example, an alkyl haloformate such as ethyl chloroformate and isobutyl chloroformate.

The reaction between the compound of general formula (I) or its acid addition salt and the carboxylic acid of general formula (IV) or its reactive derivative gives the compound of general formula V

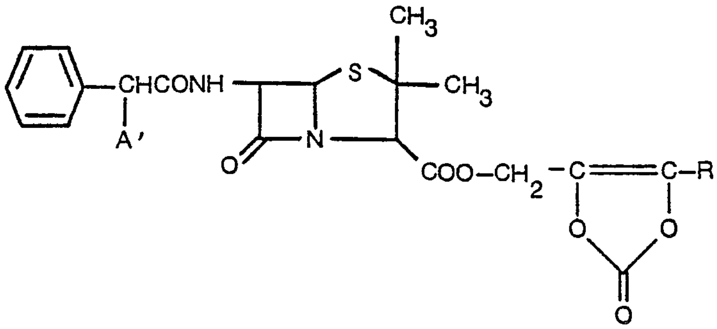

(V)

wherein R is as defined above and A′ represents a protected amino group, a group convertible to an amino group, or an amino group.

When the compound of formula V has a protected amino group or a group convertible to an amino group, the protecting group is removed from the protected amino group, or the convertible group is converted to an amino group and if desired, the product is converted to its acid addition salt. Thus, the Ampicillin ester of general formula II or its acid addition salt is formed.

According to preferred embodiments of the above process, there is provided a process for producing the Ampicillin ester of general formula II or its acid addition salt which comprises reacting a compound of general formula IV in which A is a Schiff base group or an enamine group with the compound of general formula I, thereafter converting the Schiff base group or the enamine group (A) of the resulting compound to an amino group and if required, converting the product into its acid addition salt; and a process for producing an acid addition salt, such as a hydrochloride, of the Ampicillin ester of general formula II which comprises reacting a compound of general formula IV in which A is in the form of an acid addition salt such as a hydrochloride with the compound of general formula I.

After the reaction, the Ampicillin of general formula II or its acid addition salt can be isolated and purified in a customary manner.

Specific examples of Ampicillin esters which may be produced using the inventive 6-aminopenicillanic acid ester I are

Ampicillin(5-methyl-2-oxo-1,3-dioxolen-4-yl)-methyl ester (R = methyl) and Ampicillin(2-oxo-5-phenyl-1,3-dioxolen-4-yl)-methyl ester (R = phenyl).

The acid addition salts of these Ampicillin esters are, for example, salts of these esters with inorganic acids such as hydrochloric acid, hydrobromic acid, hydriodic acid and sulfuric acid, or salts of these with organic acids such as citric acid and tartaric acid.

Investigations of the present inventors have shown that the Ampicillin esters or the acid addition salts thereof have very desirable properties as pharmaceuticals.

Specifically, in oral administration, the Ampicillin esters of the invention are easily absorbed from the digestive tract, liberate Ampicillin *in vivo,* and maintain a high Ampicillin concentration in blood over long periods of time.

For example, thirty minutes after oral administration in mice, Ampicillin (5-methyl-2-oxo-1,3-dioxolen-4-yl)methyl ester hydrochloride and Ampicillin (2-oxo-5-phenyl-1,3-dioxolen-4-yl)methyl ester hydrochloride show an Ampicillin concentration in blood about 3 times as high as that attained by the administration of Ampicillin trihydrate and about 1.5 times as high as that attained by the administration of Ampicillin phthalidyl ester hydrochloride, and the high Ampicillin concentrations in blood are maintained over a long period of time (see Experiment 1 given hereinbelow).

Such an excellent advantage of the Ampicillin esters of this invention is believed to be due to the fact that while these Ampicillin esters readily undergo enzymatic hydrolysis *in vivo,* they have resistance to hydrolysis in gastric and intestinal juices.

The rates of hydrolysis of Ampicillin (5-methyl-2-oxo-1,3-dioxolen-4-yl)methyl ester hydrochloride and Ampicillin (2-oxo-5-phenyl-1,3-dioxolen-4-yl)methyl ester hydrochloride in simulated gastric and intestinal juices are about one half of that of Ampicillin phthalidyl ester (see Experiment 2, (a) and (b) hereinbelow).

Needless to say, this high chemical stability of the penicillin esters of the invention is very beneficial not only in bulk preparation and pharmaceutical preparation and also in actual administration.

It is also noted that the Ampicillin esters of the invention have low toxicity (see Experiment 3 hereinbelow).

Experiments 1 to 3 are described below for demonstrating these advantages of these Ampicillin esters.

Experiment 1

Concentration in blood in oral administration

Test Compounds:

A. Ampicillin (5-methyl-2-oxo-1,3-dioxolen-4-yl)methyl ester hydrochloride (compound of the invention)

B. Ampicillin (2-oxo-5-phenyl-1,3-dioxolen-4-yl)methyl ester hydrochloride (compound of the invention)

C. Ampicillin phthalidyl ester hydrochloride (a known compound used as a control; see GB—A—1,364,672)

D. Ampicillin trihydrate (control)

Each of the test compounds was orally administered in a dose of 50 mg/kg calculated as Ampicillin to four week old mice (ddY, body weight about 20 g, five per group) which had been caused to fast overnight (the amount is equivalent to 0.2 ml of a 5 mg/ml aqueous solution of Ampicillin). The blood was taken from the experimental animals periodically, and the concentration of Ampicillin in the serum was measured by a bioassay method. The blood Ampicillin level ratio was calculated from the following equation.

$$\text{Ampicillin level ratio} = \frac{\text{Serum Ampicillin level in administration of each of the compounds A, B, C and D}}{\text{Serum Ampicillin level in administration of the compound D}}$$

TABLE 1

| Test compound (Item / Time of blood taking (min.)) | | Ampicillin level ratio 15 | 30 | 60 | 90 | 120 | 180 |
|---|---|---|---|---|---|---|---|
| Compounds of the invention | A | 2.8 | 2.9 | 2.1 | 1.8 | 1.5 | 1.3 |
| | B | 2.4 | 2.8 | 2.4 | 1.9 | 1.2 | 1.0 |
| Known compound | C | 3.0 | 1.8 | 1.4 | 1.1 | 0.9 | 0.8 |
| Control compound | D | 1.0 | 1.0 | 1.0 | 1.0 | 1.0 | 1.0 |

The results given in Table 1 clearly show that the compounds of the invention show a high blood Ampicillin level over a longer period of time than the known phthalidyl ester C.

Experiment 2

Hydrolyzability in acidic and basic media

(a) Hydrolyzability in an acidic medium

Test compounds:

Compounds A, B and C (see Experiment 1)

Each of the test compounds was dissolved to a predetermined concentration in an acidic aqueous medium (simulated gastric juice) having a pH of 1.2 prepared by adding 2.0 g of sodium chloride, 24 ml of 10% hydrochloric acid and 3.2 g of pepsin to 1000 ml of water. While the solution was shaken at 37°C, it was periodically sampled. The sampled solution was subjected to high-speed liquid chromatography using a reversed phase partition column, and the hydrolysis ratio of the compound was determined from a decrease in the peak height of the compound in the chromatogram.

TABLE 2

| Test compound \ Item: Time of sampling (hrs) | | Hydrolysis ratio (%) | | | | |
|---|---|---|---|---|---|---|
| | | 1 | 2 | 4 | 6 | 20 |
| Compounds of the invention | A | 7 | 15 | 20 | 28 | 52 |
| | B | 9 | 20 | 30 | 35 | 65 |
| Known compound | C | 18 | 31 | 43 | 55 | 100 |

(b) Hydrolyzability in a basic medium
Test compounds:
Compounds A, B and C (see Experiment 1)
The procedure of (a) was repeated except that a basic aqueous medium (simulated intestinal juice) having a pH of 7.50 prepared by adding 35.8 g of disodium phosphate. 6.0 ml of 10% hydrochloric acid and 2.8 g of pancreatin to 1000 ml of water was used instead of the acidic aqueous medium.

TABLE 3

| Test compound \ Item: Time of sampling (min.) | | Hydrolysis ratio (%) | | | | |
|---|---|---|---|---|---|---|
| | | 5 | 10 | 20 | 30 | 60 |
| Compounds of the invention | A | 16 | 32 | 48 | 65 | 80 |
| | B | 18 | 37 | 53 | 70 | 90 |
| Known compound | C | 39 | 61 | 90 | 95 | 100 |

The results given in Tables 2 and 3 demonstrate that the compounds of this invention have higher chemical stability in acidic and basic conditions than the known phthalidyl ester C.

Experiment 3

Acute toxicity:—
The acute toxicity values ($LD_{50}$) of the compounds A and B in Experiment 1 administered as an aqueous solution were measured using the same ddY mice as used in Experiment 1. The results are shown in Table 4. The results show that the compounds of this invention have low toxicity.

TABLE 4

| Test compound \ Route \ Item | LD$_{50}$ (mg/kg) | | |
|---|---|---|---|
| | oral | Intraperitoneal | Intravenous |
| A | >5,000 | 1,430 | 557 |
| B | >5,000 | 1,768 | 270 |

Prodrugs such as Ampicillin pivaloyloxymethyl ester or Ampicillin phthalidyl ester have been known as orally administrable Ampicillin. The ester group of the Ampicillin ester of the invention is shown by a formula below in comparison with those of the known prodrugs.

| | Ester group |
|---|---|
| Ampicillin pivaloyloxy methyl ester | 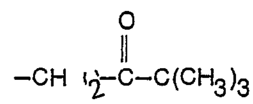 |
| Ampicillin phthalidyl ester | 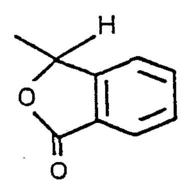 |
| Ampicillin ester of the invention | 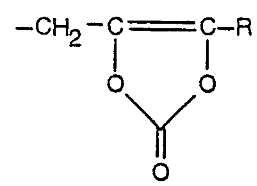 |

It is clear therefore that the ester group of this Ampicillin ester quite differs from those of the known Ampicillin esters. It is surprising that these Ampicillin esters have the aforesaid excellent properties as pharmaceuticals over the known Ampicillin esters.

The Ampicillin ester of general formula II or its pharmaceutically acceptable acid addition salt is converted back to Ampicillin *in vivo* when administered to an animal.

Example 1

Production of (5-methyl-2-oxo-1,3-dioxolen-4-yl)-methyl 6 aminopenicillanate hydrochloride:

(1) Production of 4-bromomethyl-5-methyl-1,3-dioxolen-2-one:

In 150 ml of carbon tetrachloride was dissolved 3.42 g of 4,5-dimethyl-1,3-dioxolen-2-one (synthesized by the method described in Tetrahedron Letters, 1972, pages 1701—1704). N-bromosuccinimide (5.34 g) and a catalytic amount of $\alpha,\alpha'$-azobisisobutyronitrile were added to the solution, and the mixture was heated under reflux for 15 minutes. The reaction mixture was concentred to one half of its volume, and the insoluble material was removed by filtration. The filtrate was concentrated, and the syrupy residue was distilled under reduced pressure to give 4.2 g (yield 73%) of a colorless liquid having a boiling point of 115—120°C/5 mm. The product had the following properties.

Elemental analysis, molecular formula $C_5H_5BrO_3$:
Calculated (%): C, 31.12; H, 2.61; Br, 41.40
Found (%): C, 31.30; H, 2.49; Br, 41.31

NMR ($CCl_4$, δ(ppm)):
2.10 ($-CH_3$, s), 4.10 ($-CH_2Br$, s).

From these data, the product was identified as the title compound.

(2) Production of Benzylpenicillin (5-methyl-2-oxo-1,3-dioxolen-4-yl)methyl ester:

Ten grams of benzylpenicillin potassium salt was dispersed in 50 ml of dimethylformamide, and with ice cooling, 520 mg of potassium hydrogen carbonate and 5.2 g of 5-bromomethyl-5-methyl-1,3-dixolen-2-one were added, then the mixture was stirred at 0°C for 4 hours. The reaction mixture was poured into ice water, and the precipitated solid was collected by filtration. It was dissolved in ethyl acetate, washed with a dilute aqueous solution of sodium hydrogen carbonate and then repeatedly with ice water. The ethyl acetate layer was then dried over anhydrous magnesium sulfate, and the solvent was distilled off under reduced pressure. There was obtained 12.5 g (yield 94%) of benzylpenicillin (5-methyl-2-oxo-1,3-dioxolen-4-yl)methyl ester.

IR (KBr): 1825 $cm^{-1}$ (cyclic carbonate), 1785 $cm^{-1}$ (β-lactam), 1750 $cm^{-1}$ (ester), 1670 $cm^{-1}$ (amide)

NMR ($CDCl_3$ δ(ppm)):
1.37 and 1.42 (6H, methyl at the 2-position, s), 2.13

(3H, 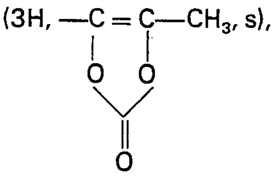 , s),

3.72 (2H, $-CH_2-C_6H_5$, s) 4.29 (1H, proton at the 3-position, s), 4.80

(2H, 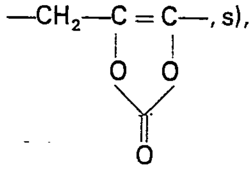 , s),

(2H, protons at the 5- and 6- positions, m), 6.16 (1H, NH, d), 7.14 (5H, protons on the benzene ring, s).

(3) Production of (5-methyl-2-oxo-1,3-dioxolen-4-yl)-methyl 6-aminopenicillanate hydrochloride:

Phosphorus pentachloride (5.9 g) was dissolved in dry methylene chloride (30 ml), and 6.3 ml of quinoline was added. The solution was cooled to −30°C with dry ice-acetone. With vigorous stirring, 11 g of the above benzylpenicillin (2-oxo-5-methyl-1,3-dioxolen-4-yl)methyl ester dissolved in dry methylene chloride (10 ml) was added dropwise, and the mixture was stirred at this temperature for 35 minutes. Propyl alcohol (20 ml) was added dropwise over 5 minutes, and the mixture was stirred for 30 minutes. With vigorous stirring, 20 ml of a saturated solution of sodium chloride was added dropwise and the mixture was stirred for about an hour. Then, the methylene chloride layer was separated, washed with saturated sodium chloride solution, dried over anhydrous sodium sulfate and evaporated to dryness to afford a gum. The gum was washed with n-hexane to give (5-methyl-2-oxo-1,3-dioxolen-4-yl)methyl 6-amino-penicillanate hydrochloride as a pale yellow amorphous substance.

Melting point: 115—120°C (decomp.)

Elemental analysis, molecular formula $C_{13}H_{16}N_2O_6S \cdot HCl \cdot \frac{1}{2}H_2O$:
Calculated (%): C, 41.77; H, 4.85; N, 7.49
Found (%): C, 41.91; H, 4.77; N, 7.67

IR(KBr): 1825 $cm^{-1}$ (cyclic carbonate), 1790 $cm^{-1}$ (β-lactam), 1750 $cm^{-1}$ (ester).

NMR(DMSO—$d_6$, $\delta$ppm)):

1.45 and 1.66 (6H, methyl at the 2-position, s) 2.22

(3H, 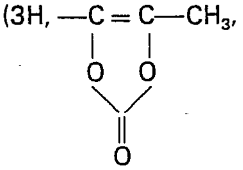 s),

4.57 (1H, proton at the 3-position, s), 5.0—5.2

(3H, proton at the 6-position, 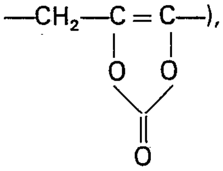),

5.56 (1H, proton at the 5-position, d)

Example 2

Production of (2-oxo-5-phenyl-1,3-dioxolen-4-yl)-methyl-6-aminopenicillanate hydrochloride:

(1) Production of 4-bromomethyl-5-phenyl-1,3-dioxolen-2-one:

In 150 ml of carbon tetrachloride was dissolved 2.4 g of 4-methyl-5-phenyl-1,3-dioxolen-2-one (synthesized by the method described in Liebichs Annalen der Chemie, Vol. 764, pages 116—124, 1972). N-bromosuccinimide (2.9 g) and a catalytic amount of $\alpha,\alpha'$-azobisisobutyronitrile were added to the solution, and the mixture was heated under reflux for 90 minutes. The reaction mixture was concentrated to one half of its volume, and the insoluble material was separated by filtration. The filtrate was concentrated, and the residue was recrystallized from a mixture of benzene and cyclohexane to give 2.3 g (yield 66%) of colorless needles having a melting point of 90.5 to 91.5°C. This product had the following properties.

Elemental analysis, molecular formula $C_{10}H_7BrO_3$:

Calculated (%): C, 47.09; H, 2.77; Br, 31.33

Found (%): C, 47.22; H, 2.64; Br, 31.29

NMR ($CCl_4$, $\delta$(ppm)):

4.35 (—$CH_2Br$, s), 7.40 (benzene ring, s).

From these data, the product was identified as the title compound.

(2) Production of (2-oxo-5-phenyl-1,3-dioxolen-4-yl)methyl 6-aminopenicillanate hydrochloride:

By the same method as shown in Example 1, ((2) (3)) (2-oxo-5-phenyl-1,3-dioxolen-4-yl)methyl 6-aminopenicillanate hydrochloride was obtained in a yield of 74% from 4-bromomethyl-5-phenyl-1,3-dioxolen-2-one and benzylpenicillin potassium salt.

Example 3

Production of (5-methyl-2-oxo-1,3-dioxolen-4-yl)methyl 6-aminopenicillanate p-toluenesulfonate:

In 100 ml of dimethylformamide was dissolved 13 g of 6$\beta$-tritylaminopenicillanic acid synthesized by the method described in J. Am. Chem. Soc. *84,* 2983 (1963). The solution was cooled to 0 to 5°C, and 3 g of potassium hydrogen carbonate and 6 g of 4-bromomethyl-5-methyl-1,3-dioxolen-2-one were added. The mixture was stirred at the above temperature for 3 hours. After the reaction, the reaction mixture was poured into ice water. The precipitated yellow solid was extracted with 300 ml of ethyl acetate. The ethyl acetate layer was washed several times with a saturated aqueous solution of sodium chloride, dried over anhydrous magnesium sulfate and concentrated under reduced pressure to give a yellow syrup. The syrup was dissolved in 80 ml of ethyl acetate, and with ice cooling, 5.2 g of p-toluenesulfonic acid was added. The mixture was stirred under ice cooling for 1 hour, whereupon a colorless solid precipitated. The solid was collected by filtration and well washed with ethyl acetate to give 8.3 g (yield 60%) of the title compound.

IR (KBr): 1820 $cm^{-1}$ (cyclic carbonate), 1780 $cm^{-1}$ ($\beta$-lactam), 1760 $cm^{-1}$ (ester)

NMR (DMSO—$d_6$, $\delta$(ppm)):

1.40 and 1.59 (6H, methyl at the 2-position, s), 2.12

(3H, 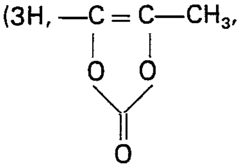, s),

4.46 (1H, proton at the 3-position, s), 4.90—5.10

(3H, proton at the 6-position and 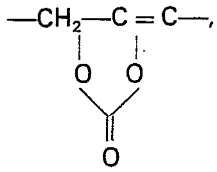, m),

5.41 (1H,

(proton at the 5-position, d), 2.24 (3H,

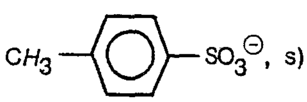, s), 6.97 and 7.38 (4H, aromatic

protons of 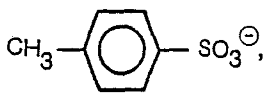, d).

## Example 4

Production of Ampicillin (5-methyl-2-oxo-1,3-dioxolen-4-yl)methyl ester hydrochloride:

(1) Production of D-(—)-phenylglycyl chloride hydrochloride:

Separately, 10 g of D-(—)-phenylglycine was added to 250 ml of methylene chloride. The mixture was cooled to 0°C, and by passing hydrogen chloride gas, the hydrochloride of the phenylglycine was formed. Phosphorus pentachloride (20 g) was added, and the mixture was stirred at 0 to 5°C for 4 hours. The solid precipitated was collected by filtration, and repeatedly washed with methylene chloride to give 13.1 g (yield 90%) of D-(—)-phenylglycyl chloride hydrochloride as a colorless amorphous solid.

(2) Production of Ampicillin (5-methyl-2-oxo-1,3-dioxolen-4-yl)methyl ester hydrochloride:

200 milligrams of the 6-aminopenicillanic acid ester hydrochloride obtained in Example 1 (3) was dispersed in 10 ml of methylene chloride, and 50 mg of potassium hydrogen carbonate was added. The mixture was stirred at 0°C for 15 minutes. Then, 110 mg of the acid chloride obtained as above was added, and the mixture was stirred for 2 hours and then for another 2 hours at room temperature.

After the reaction, the solid was separated by filtration, and the filtrate was concentrated under reduced pressure. The resulting syrup was dissolved in water, and washed with ethyl acetate. The aqueous layer was saturated with sodium chloride, and the separated oily substance was extracted with methylene chloride. The extract was washed with a saturated aqueous solution of sodium chloride and concentrated until the amount of methylene chloride decreased to half. Upon addition of isopropyl alcohol, a colorless solid was precipitated. The solid was collected by filtration and washed with isopropyl alcohol and ether to give 132 mg (yield 54%) of Ampicillin (5-methyl-2-oxo-1,3-dioxolen-4-yl)methyl ester hydrochloride as an amorphous solid.

The product had the following properties.

Appearance: Colorless amorphous solid

Melting point: 145°C (decomp.)

Elemental analysis, molecular formula $C_{21}H_{23}N_3O_7S{\cdot}HCl{\cdot}H_2O$:

Calculated (%): C, 48.88; H, 5.08; N, 8.14; S, 6.21

Found (%): C, 48.51; H, 5.15; N, 8.02; S, 6.44

IR (KBr): 1825 $cm^{-1}$ (cyclic carbonate), 1785 $cm^{-1}$ ($\beta$-lactam), 1750 $cm^{-1}$ (ester), 1690 $cm^{-1}$) (amide).

NMR (DMSO—$d_6$, $\delta$(ppm)):
1.33 and 1.48 (6H, methyl at the 2-position, s), 2.19

3H, 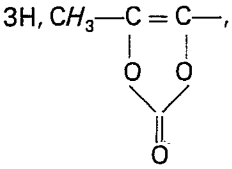 , s),

4.43 (1H, proton at the 3-position, s), 5.11

(2H, 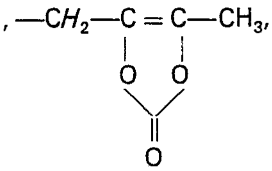 , s),

5.16 (1H, benzyl proton, s), 5.43—5.65 (2H, protons at the 5- and 6-positions, m), 7.3—7.6 (5H, protons on the benzene ring, m). 8.95 (3H, —$\overset{\oplus}{N}H_3$), 9.4 (1H, CONH—, d).

The resulting Ampicillin ester hydrochloride was incubated in 40% mouse blood in pH 7.4 phosphate buffer at 37°C for 10 minutes, and then subjected to bioautography. It was found to be completely converted to Ampicillin.

Example 5

Production of Ampicillin (2-oxo-5-phenyl-1,3-dioxolen-4-yl)methyl ester hydrochloride:

By the same method as shown in Example 4, the title compound was obtained from D-(—)-phenylglycyl chloride hydrochloride and (2-oxo-5-phenyl-1,3-dioxolen-4-yl)methyl 6-aminopenicillanate hydrochloride as a colorless amorphous solid.

The product had the following properties.

Melting point: 140°C (decomp.)

Elemental analysis, molecular formula $C_{26}H_{25}N_3O_7S{\cdot}HCl{\cdot}2H_2O$:
Calculated (%): C, 52.39; H, 5.07; N, 7.05; S, 5.38
Found (%): C, 52.17; H, 4.83; N, 7.31; S, 5.64

IR (KBr): 1830 $cm^{-1}$ (cyclic carbonate), 1785 $cm^{-1}$ ($\beta$-lactam), 1760 $cm^{-1}$ (ester), 1690 $cm^{-1}$ (amide)

NMR (DMSO—$d_6$, $\delta$(ppm)):
1.32 and 1.45 (6H, methyl at the 2-position, s), 4.44 (1H, proton at the 3-position, s), 5.12 (1H, benzyl proton, s), 5.31

(2H, 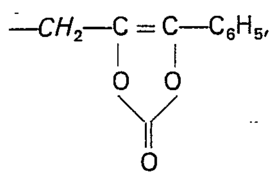 , s),

5.4—5.6 (2H, protons at the 5- and 6-positions, m), 7.3—7.6 (10H, protons on the benzene ring, m), 8.8 (3H, —$\overset{\oplus}{N}H_3$), 9.3 (1H, —CONH—, d).

The resulting Ampicillin ester hydrochloride was incubated in 40% mouse blood in pH 7.4 phosphate buffer at 37°C for 10 minutes, and then subjected to bioautography. It was found to be completely converted to Ampicillin.

**Claims**

1. A 6-aminopenicillanic acid ester of the general formula I

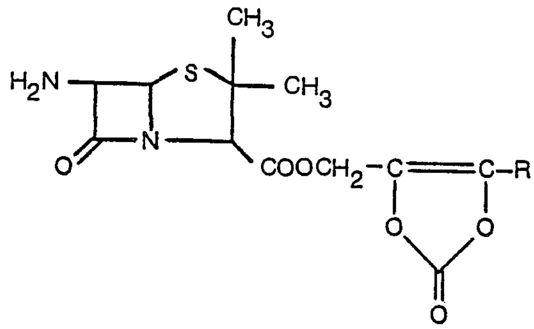

(I)

wherein R represents a methyl group or a phenyl group, or its acid addition salt.

2. A process for producing a 6-aminopenicillanic acid ester of claim 1 characterized by reacting 6-aminopenicillanic acid or its salt at the carboxyl group with a compound of the general formula III

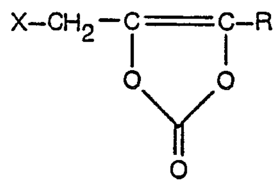

(III)

wherein R is as defined in claim 1 and X represents a halogen atom; or by reacting 6-protected aminopenicillanic acid or its salt at the carboxyl group with the compound of general formula III and then converting the protected amino group of the reaction product to an amino group.

3. Use of the 6-aminopenicillanic acid ester of claim 1 for the production of an Ampicillin ester of the general formula II

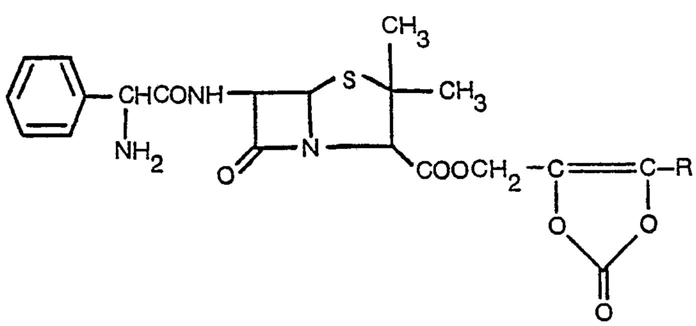

(II)

wherein R represents a methyl group or a phenyl group or its acid addition salt.

**Patentansprüche**

1. 6-Aminopenicillansäureester der allgemeinen Formel I:

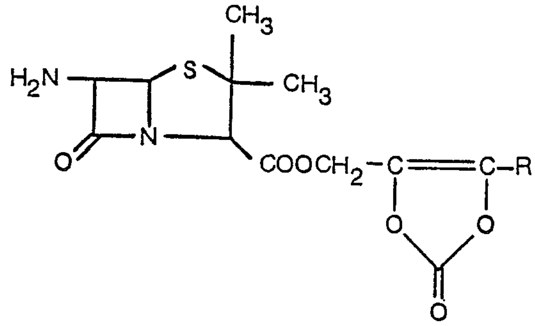

(I)

worin R für eine Methylgruppe oder eine Phenylgruppe steht, oder sein Säureadditionssalz.

2. Verfahren zur Herstellung eines 6-Aminopenicillansäureesters nach Anspruch 1, dadurch gekennzeichnet, daß man 6-Aminopenicillansäure oder ihr Salz an der Carboxylgruppe mit einer Verbindung der allgemeinen Formel III:

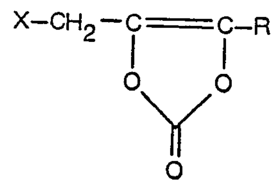

(III)

worin R wie im Anspruch 1 definiert ist und X für ein Halogenatom steht, umsetzt oder indem man 6-geschützte Aminopenicillansäure oder ihr Salz an der Carboxylgruppe mit der Verbindung der allgemeinen Formel III umsetzt und sodann die geschützte Aminogruppe des Reaktionsprodukts in eine Aminogruppe umwandelt.

3. Verwendung des 6-Aminopenicillansäureesters nach Anspruch 1 zur Herstellung eines Ampicillinesters der allgemeinen Formel II:

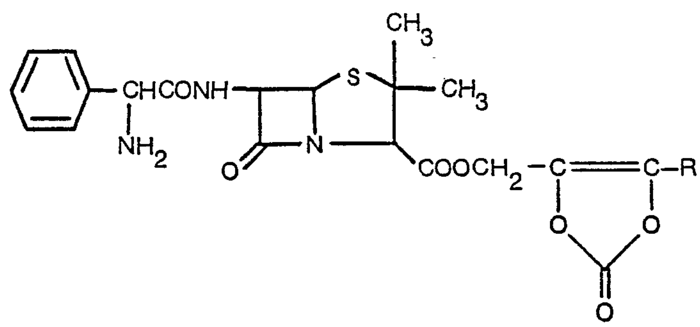

(II)

worin R für eine Methylgruppe oder eine Phenylgruppe steht, oder seines Säureadditionssalzes.

**Revendications**

1. Esters de l'acide 6-aminopénicillanique de formule générale I ci-dessous:

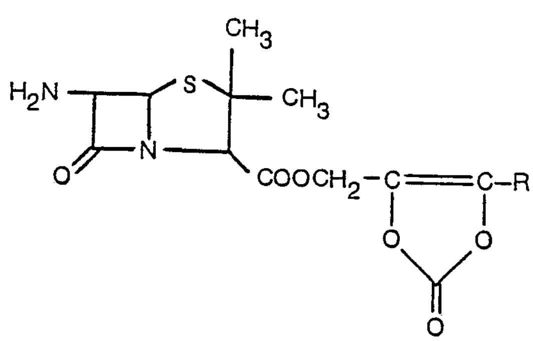

(I)

le symbole R représentant un groupe méthyle ou phényle, ainsi que leurs sels d'addition d'acides.

2. Un procédé de préparation des esters de l'acide 6-aminopénicillanique selon la revendication 1, procédé caractérisé en ce que l'on fait réagir l'acide 6-aminopénicillanique ou un sel de son groupe carboxylique avec un composé de formule générale III:

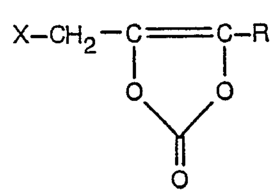

(III)

R ayant la signification donné à la revendication 1 et X désignant un atome d'halogène; ou bien on fait réagir l'acide 6-aminopénicillanique dont le groupe amino est protégé, ou un sel de son

groupe carboxylique, avec le composé de formule III, puis on refait passer à l'état de groupe amino libre le groupe amino protégé du produit formé.

3. Utilisation des esters de l'acide 6-aminopénicillanique selon la revendication 1 pour la production d'esters d'ampicilline de formule générale II:

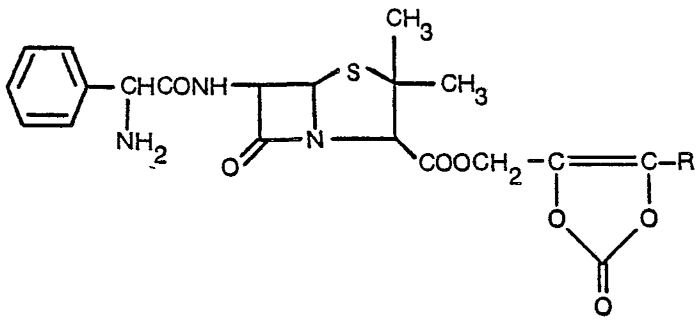

(II)

le symbole R désignant un groupe méthyle ou phényle, ainsi que de leurs sels d'addition d'acides.